# EUROPEAN PATENT SPECIFICATION

(11) **EP 4 452 066 B1**
(45) Date of publication and mention of the grant of the patent: **11.11.2026**
(21) Application number: 22822653.6
(22) Date of filing: 20.11.2022
(51) Int. Cl.: A61B 5/287, A61B 5/00

(54) **ELECTROPHYSIOLOGICAL (EP) MAP POINTS ADJUSTMENTS BASED ON USER CLINICAL INTERPRETATION**
ANPASSUNGEN VON ELEKTROPHYSIOLOGISCHEN KARTENPUNKTEN AUF BASIS KLINISCHER INTERPRETATIONEN VON BENUTZERN
AJUSTEMENTS DE POINTS DE CARTE ÉLECTROPHYSIOLOGIQUE (EP) SUR LA BASE DE L'INTERPRÉTATION CLINIQUE D'UN UTILISATEUR

(30) Priority: 20.12.2021 US 202117555629
(43) Date of publication of application: 30.10.2024
(73) Proprietor: Biosense Webster (Israel) Ltd., 2066717 Yokneam (IL)
(72) Inventor: MASSARWA, Fady, 2066717 Yokneam (IL); SEGEV, Meytal, 2066717 Yokneam (IL); ALTMAN, Sigal, 2066717 Yokneam (IL)
(74) Representative: Carpmaels & Ransford LLP
(86) International application number: PCT/IB2022/061184
(87) International publication number: WO 2023/119005

(56) References cited:
- US-A1- 2012 035 488
- US-A1- 2018 296 108
- US-A1- 2021 391 082

## Description

### FIELD OF THE DISCLOSURE

The present disclosure relates generally to electrophysiological mapping, and particularly to manually-assisted editing of cardiac electrophysiological maps.

### BACKGROUND OF THE DISCLOSURE

Editing tools for assisting in the interpretation of an electrophysiological map were previously proposed in the patent literature. For example, U.S. Patent No. 8,478,393 describes a method for visualization of electrophysiology data representing electrical activity on a surface of an organ over a time period. An interval within the time period is selected in response to a user selection. Responsive to the user selection of the interval, a visual representation of physiological information for the user selected interval is generated by applying at least one method to the data. The visual representation is spatially represented on a graphical representation of a predetermined region of the surface of the organ.

In US2018296108A1, there are described systems and methods for facilitating processing of cardiac information based on sensed electrical signals, where the systems include a processing unit configured to receive a set of electrical signals; receive an indication of a measurement location corresponding to each electrical signal of the set of electrical signals; and generate, based on at least one of an annotation waveform corresponding to each electrical signal of the set of electrical signals and a set of annotation mapping values, an annotation histogram.

In US2012035488A1, there is described an electrophysiology (EP) system that includes an interface for operator-interaction with the results of code executing therein. A template model can have channels positioned or repositioned thereupon by the user to define a set-up useful in rapid catheter positioning. Mapping operations can be performed without the requirement for precise catheter location determinations. A map module coordinates EP data associated with each selected channel and its associated position on the template model to provide this result, and can update the resulting map in the event that the channel or location is changed. Messaging and other dynamic features enable synchronized presentation of a myriad of EP data. Additional systems and methods are disclosed herein.

In US2021391082A1, there is described a method that includes modeling a vector velocity field that measures and quantifies a velocity of electrocardiogram data signals that pass through a local activation time. The method further includes determining codes for each point in plane to provide a color code vector field image; detecting focal and rotor indications by using kernels to scan the color-coded vector field image; and classifying the focal and rotor indications into perpetuators.

### BRIEF DESCRIPTION OF THE DRAWINGS

The present disclosure will be more fully understood from the following detailed description of the examples thereof, taken together with the drawings in which:
Fig. 1 is a schematic, pictorial illustration of a system for electrophysiological (EP) mapping, in accordance with an example of the present disclosure;
Figs. 2A-2C are schematic, pictorial EP maps overlayed with user clinical inputs of propagation paths (2A and 2B) and region marking (2C), in accordance with examples of the present disclosure;
Fig. 3 is a schematic drawing of a hinted activation path provided by a user as clinical input, along with annotation times recalculated to be consistent with the hinted path, in accordance with an example of the present disclosure;
Figs. 4A-4C are schematic illustrations of steps in the recalculation of annotation times shown in Fig. 3, to be consistent with the hinted path, in accordance with an example of the present disclosure; and
Fig. 5 is a flow chart that schematically illustrates a method for adjustment of data points of an EP map using user clinical input, in accordance with another example of the present disclosure.

### DETAILED DESCRIPTION OF EXAMPLES

### OVERVIEW

Catheter-based electrophysiological (EP) mapping techniques may produce various types of EP maps of an organ, such as a left atrium of a heart. Cardiac EP maps, such as a local activation time (LAT) map, a bipolar potential map, or a unipolar potential map, are produced by acquiring electrograms from locations on a heart chamber surface. EP values, such as LATs (or potentials), are then derived from the electrograms for the locations. Such locations and respective EP values, called hereafter "data points," are then overlayed, typically using colors, onto a 3D map of the chamber.

In practice, analysis of the vast number of data points that are acquired may lead to erroneous results in an EP map. Errors in EP maps are due to various difficulties, such as during acquisition (e.g., low signal to noise ratio, mechanical distortion of a cardiac wall by a catheter), and in the analysis stages (e.g., erroneous time annotations of activations).

For example, some of the LAT annotations may become inaccurate in complicated algorithms. To mitigate inaccuracies, an LAT consistency algorithm may be used to identify inaccurate LATs, and, once identified, an inaccurate LAT is not used to color the map. LAT corrections may be based on altering a window of interest (WOI - a portion of the cardiac cycle used for LAT estimation) over the EP signal and/or adjusting a threshold in the LAT consistency algorithm. Still, none of the above methods prevent incorporating outlier EP values into an EP map.

Therefore, typically, the physician often corrects inaccuracies manually when observed. Such manual correction by the physician is tedious and time consuming. Moreover, as the number of acquired data points increases with modern multi-electrode catheters, it becomes increasingly impractical for a physician to perform manual correction.

Examples of the present disclosure that are described hereinafter provide methods and systems that utilize clinical input provided by the physician to improve the accuracy in a specific portion of an EP map. For example, clinical input from an experienced physician can be used to automate the corrections that would typically be made manually by the same physician. Rather than having the physician enter pinpointed manual corrections, the disclosed technique relies instead on high-level insights made by the physician, typically by letting the physician enter certain general clinically meaningful tendencies on the EP map (e.g., draw on a touchscreen displaying the map), and then automatically adjusting the map so that the points are consistent with these observed "global" tendencies.

In one example, the physician may draw a directional curve showing a clinically observed direction of EP wave propagation. The automatically calculated LATs along that arrow may be recomputed to provide more accurate LATs based on this additional input. The location and direction of the arrow may be used by the disclosed technique and algorithm to improve the LAT map in areas that are clinically critical.

Improved accuracy may be attained, for example, by moving the WOI or by defining a smaller WOI for determining LAT. The new WOI may be determined based on the direction of the arrow provided by the physician as well as neighboring LATs. The direction of the arrow may also provide input to the LAT consistency algorithm so that the clinical input is considered when selecting outliers.

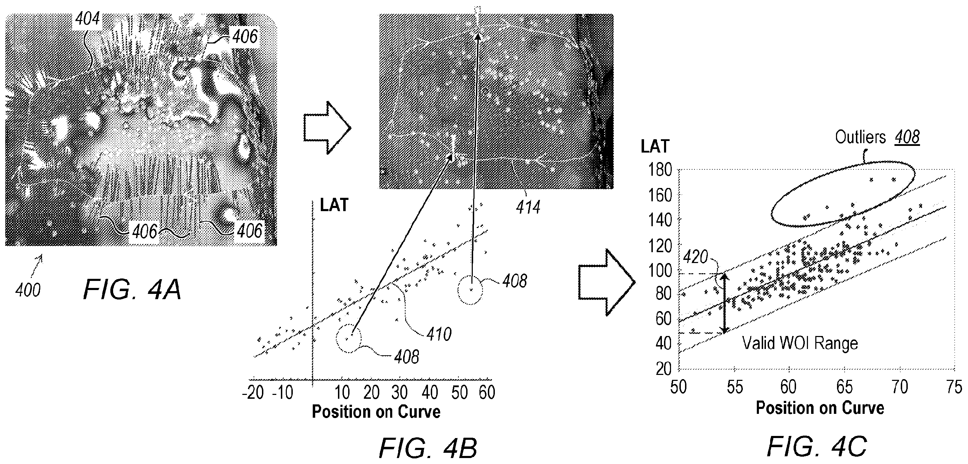

In an example, a processor receives an EP map with user clinical input in a form of hint-activation paths. The processor sorts the data points (each data point made of an EP value at a position on the EP map, as shown in Fig. 4A). Then, the processor segments the path by running piecewise regression to find a best fitting LAT activation path. The processor adjusts WOI for each data point and uses the adjusted WOI recomputed annotations (e.g., LAT annotations) to generate a more accurate EP map and, importantly, one that is consistent with the clinical input.

In another example, a physician may circle an area that is clinically observed to have a specific characteristic. This clinical input may be used to improve the mapping. For example, the sensitivity of the LAT consistency algorithm may be adjusted based on the classification of the region. In one example, the characteristic is scarred tissue. In this case, points inside the circled area may not be classified as outliers. By automating EP map editing based on user insights that are given in the form of informal drawings on the map, the technique improves map quality of multi-electrode catheter systems that acquire a vast number of data points in a short period of time.

Typically, the processor is programmed in software containing a particular algorithm that enables the processor to conduct each of the processor-related steps and functions outlined above.

By increasing EP map accuracy using the aforementioned interactive graphical means provided to the physician, and an algorithm to implement a physician's informal (e.g., hand drawn) inputs, the disclosed techniques may assist the physician in the interpretation of EP maps and thus expedite and improve the quality of complicated diagnostic tasks, such as those required in diagnostic catheterizations.

### SYSTEM DESCRIPTION

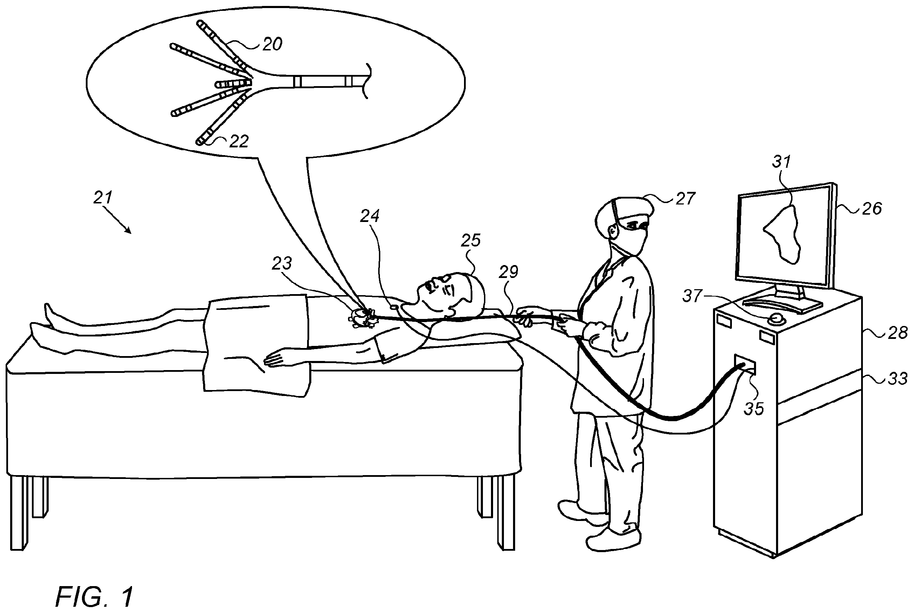

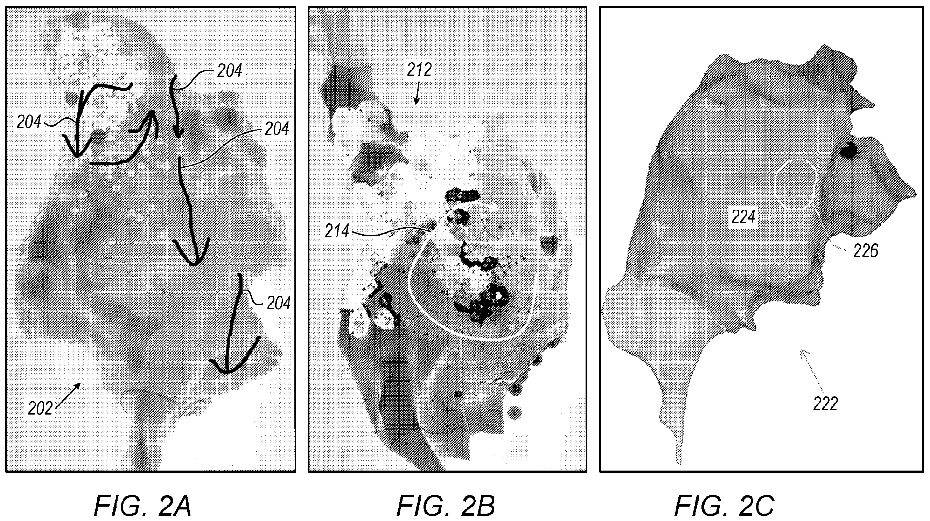

Fig. 1 is a schematic, pictorial illustration of a system 21 for electrophysiological (EP) mapping, in accordance with an example of the present disclosure. Fig. 1 depicts a physician 27 using a mapping Pentaray^{®} catheter 29 to perform an EP mapping of a heart 23 of a patient 25. Catheter 29 comprises, at its distal end, one or more arms 20, which may be mechanically flexible, each of which is coupled with one or more electrodes 22. During the mapping procedure, electrodes 22 acquire and/or inject unipolar and/or bipolar signals from and/or to the tissue of heart 23. A processor 28 receives these signals via an electrical interface 35, and uses information contained in these signals to construct an EP map 31 stored by processor 28 in a memory 33. During and/or following the procedure, processor 28 may display EP map 31 on a display 26, wherein display 26 can be a touchscreen to enable physician 27 marking clinical inputs on EP map 31, such as marking activation paths and scar regions, as shown in Figs. 2A-2C. Alternatively or additionally, physician 27 may mark the clinical inputs using any other suitable input device, e.g., in the form of a mouse or a trackball 37.

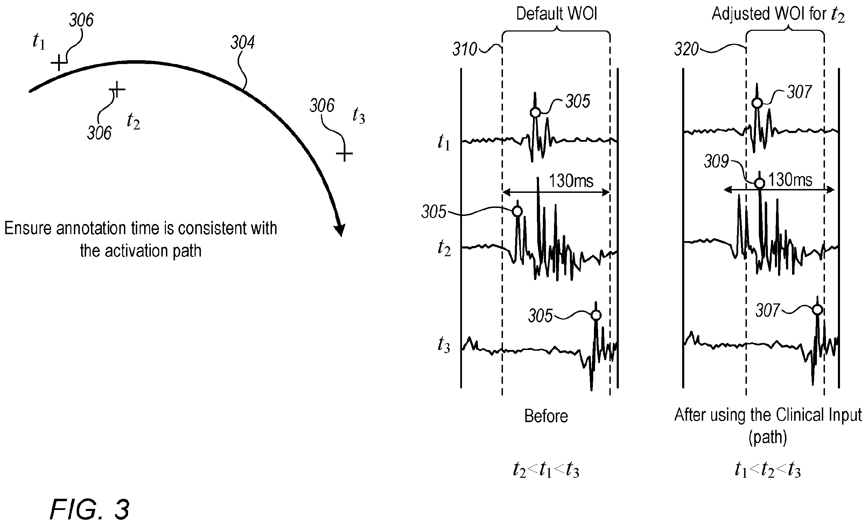

EP map 31 may be an LAT map, a bipolar potential map, or another map type. The quality of EP map 31 is improved by using the disclosed technique to derive and present a confidence level on the map, as described in Fig. 2 and Fig. 3.

During the procedure, a tracking system is used to track the respective locations of sensing electrodes 22, such that each of the signals may be associated with the location at which the signal was acquired. For example, the Active Catheter Location (ACL) system, made by Biosense-Webster (Irvine California), which is described in US Patent No. 8,456,182, may be used. In the ACL system, a processor estimates the respective locations of the electrodes based on impedances measured between each of the sensing-electrodes 22, and a plurality of surface electrodes 24 that are coupled to the skin of patient 25. For example, three surface electrodes 24 may be coupled to the patient's chest and another three surface electrodes may be coupled to the patient's back. For ease of illustration, only one surface electrode is shown in Fig. 1. Electric currents are passed between electrodes 22 inside heart 23 of the patient and surface-electrodes 24. Processor 28 calculates an estimated location of all electrodes 22 within the patient's heart based on the ratios between the resulting current amplitudes measured at surface electrodes 24 (or between the impedances implied by these amplitudes) and the known positions of electrodes 24 on the patient's body. The processor may thus associate any given impedance signal received from electrodes 22 with the location at which the signal was acquired.

The example illustration shown in Fig. 1 is chosen purely for the sake of conceptual clarity. Other tracking methods can be used, such as ones based on measuring voltage signals. Other types of sensing catheters, such as the Lasso^{®} Catheter (produced by Biosense Webster) or basket catheters may equivalently be employed. Physical contact sensors may be fitted at the distal end of mapping catheter 29 to estimate contact quality between each of the electrodes 22 and an inner surface of the cardiac chamber during measurement.

Processor 28 typically comprises a general-purpose computer with software programmed to carry out the functions described herein. In particular, processor 28 runs a dedicated algorithm as disclosed herein, including in Fig. 3, that enables processor 28 to perform the disclosed steps, as further described below. The software may be downloaded to the computer in electronic form, over a network, for example, or it may, alternatively or additionally, be provided and/or stored on non-transitory tangible media, such as magnetic, optical, or electronic memory.

### USER CLINICAL INPUT MARKED AN EP MAP

As noted above, some acquired point attributes, such as LAT values and filtering status (LAT consistency), are determined by mathematical algorithms that, in many cases, do not take the clinical diagnosis and observations of the physician into account. For example, in some arrhythmias the automatic computation of LAT values of the point in the reentry path are inaccurate, which may lead to misleading coloring and consistency determinations.

Typically, the user manually iterates over each one of the problematic points and fixes them manually (for example, by fixing the annotation or changing the consistency outlier classification). This process is tedious and, in case of multiple points, the user may not find them all.

Utilizing clinical physician hints, such as a general wave-propagation direction in specific areas, can be useful to automate this process and help the algorithm obtain better results. This disclosure describes how to incorporate various physician guidelines/hints that are based on a clinical understanding of the study into point-related algorithms, such as LAT consistency and map annotation algorithms.

The disclosure considers two types of clinical hints:
1. Directional. In this case, the physician outlines one or more directed curves on the map surface that should provide a hint about wave propagation direction or a line of blocks (based on user clinical observation). Using these curves, the map annotation algorithm can be improved by having a tighter WOI (or possibly a fixed WOI position) for each point which is determined by its nearest location on the curve (path). The new WOI is calculated based on the hint and the neighboring point annotation. Additionally, the directional curves can serve as an input for the current LAT consistency algorithm and thus improve the outlier decision for each point by considering clinical values and not only statistical regional values. Specifically, these curves can help to build a more reliable conduction path that is used in the second stage of an LAT consistency algorithm.
2. Regional. In this case, the clinical hint is over some area on the map surface which can be interpreted in several ways that can help classify the underlying points in this area, such as: (2a) a scar area where all the points inside are considered as a scar, and (2b) a high-level certainty area where points inside are never classified as outliers.

Figs. 2A-2C are schematic, pictorial EP maps overlayed with user clinical inputs of propagation paths (204 and 214 on Figures 2A and 2B, respectively) and region 226 marking (224 on Fig. 2C), in accordance with examples of the present disclosure.

In EP map 202 of Fig. 2A (an LAT map), a physician drew hinted activation paths 204 as a clinical input, with the expectation that the LAT values would be consistent along those paths (e.g., monotonically increasing).

Similarly, in EP map 212 of Fig. 2B, which is also an LAT map, a physician drew a hinted activation path 214 as a clinical input, with the expectation that the LAT values would also be consistent along this semicircular path (e.g., monotonically increasing).

In EP map 222 of Fig. 2C, which can be an LAT map or a potential map, a physician drew closed curve 224 to mark a hinted region 226 as a clinical input, with the expectation that region 226 is on the map surface, and may be interpreted in several ways that can help classify the underlying points in this area, such as:
- Scar area 226, where all included points are considered as a scar
- High level certainty area 226, where included data points are never classified as outliers

### USING USER CLINICAL INPUT OF HINTED ACTIVATION PATHS TO IMPROVE AN LAT MAP

Fig. 3 is a schematic drawing of a hinted activation path 304 provided by a user as clinical input along with annotation times (307, 309) recalculated to be consistent with the hinted path, in accordance with an example of the present disclosure.

As seen, there are three data points 306 in the vicinity of hinted activation path 304, where data points 306 are activation times t₁, t₂ and t₃ at respective locations over an LAT map, such as maps 202 and 212 of Fig. 2. The automatically calculated annotations 305 on the three respective electrograms, within a default WOI 310 (e.g., of time duration of 130 mSec), are inconsistent with the hinted activation path, since times are not monotonically increasing along with t₁>t₂.

Using the hinted activation path, the disclosed technique adjusts default WOI 310 into shorter WOI 320 (e.g., with time duration smaller than 130 mSec of WOI 310) and recalculates the annotations times.

As seen, two annotation times 307 are unchanged by the change in WOI. However, the erroneous annotation time 305 is correctly found by the algorithm to be annotation 309, which is consistent with the hinted activation path, as times are now monotonically increasing along the path, with t₁<t₂.

### ALGORITHM FOR IMPROVING AN LAT MAP USING USER CLINICAL INPUT OF HINTED ACTIVATION PATHS

Figs. 4A-4C are schematic illustrations of steps in the recalculation of annotations times, as shown in Fig. 3, to be consistent with the hinted path, in accordance with an example of the present disclosure.

Fig. 4A shows a hinted activation path 404 provided by a user as clinical input. Path 404 is drawn on an LAT map 400, and is generally similar to path 214 of Fig. 2B. As seen, the algorithm has used certain criteria to select (406) EP values relevant to reconsideration (e.g., recalculation of activation times) based on the hinted path. These selected points are in the vicinity of the curve (along the curve) with their distance from the hinted curve being smaller than a threshold that is defined as one of the algorithm parameters (this threshold can be preset or calculated automatically according to available data points). The selected data points are then projected to the curve and sorted according to their position in the curve (the closest to the curve beginning is the earlier in time).

As can be expected, path 404 is crude and, as shown in Fig. 4B, the algorithm uses a regression model to generate (e.g., segment (410)) an adjusted activation path (414) that is more accurate than the hinted path 404, due to path 414 being based on LAT data points selected (406) for reconsideration, though the adjustment statistically neglects outlier values 408.

The result, seen in Fig. 4C, is that for each position on curve 404, the algorithm provides a valid WOI 420, and the annotation times of the reconsidered data points are calculated (as shown in Fig. 3) based on the valid WOI, which are used to generate an EP map consistent with the user's clinical input, as annotation times (307, 309) are recalculated in Fig. 3.

### METHOD FOR IMPROVING AN EP MAP USING USER CLINICAL INPUT OF HINTED ACTIVATION PATHS

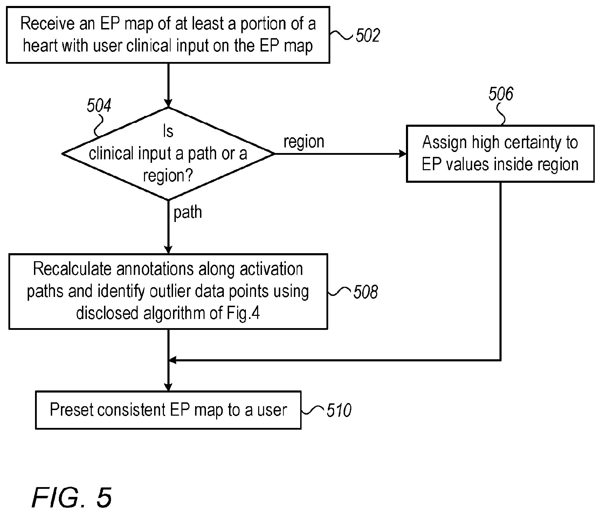

Fig. 5 is a flow chart that schematically illustrates a method for adjusting data points of an EP map using user clinical input, in accordance with another example of the present disclosure. The algorithm, according to the presented example, carries out a process that begins with processor 28 receiving an EP map (e.g., an LAT map) of at least a portion of a heart, with user clinical input on the map (e.g., a drawn hinted activation path), at a clinical input receiving step 502.

Next, the processor checks the clinical input type, if it is, for example, an activation path and/or a closed region, at a clinical input type checking step 504.

In case the clinical input is considered a region, an assigning step 506 may include an adjustment of the sensitivity of an LAT consistency algorithm based on the classification of the region. This may exclude data points inside the region from being classified as outliers. The classification is done using the WOI being centered around the regression line (at each point), and every point that is outside the updated WOI should be considered as an outlier (see Fig. 4C). In another example, all of the points inside such a region may be considered as a scar.

In case the clinical input considered is one or more hinted activation paths, step 508 may include performing the algorithm described in Fig. 4 to ensure EP map consistency along any hinted activation paths, and to identify outlier data points.

Finally, at an EP map presentation step 510, processor 28 presents the updated EP map after all clinical inputs were used in making the EP map more consistent with clinical observation by the physician. As described above, using the disclosed technique, the EP map correction is made by the physician who provides high level clinical inputs and without requiring meticulous, laborious, manual work on the side of the physician.

The example flow chart shown in Fig. 5 is chosen purely for the sake of conceptual clarity. In other examples, other types of clinical inputs may be considered, such as drawings that hint at several electro potential waves that collision at some point, or selecting a path with some thickness to indicate a scar region or slow conduction region.

It will be appreciated that the present disclosure is not limited to what has been particularly shown and described hereinabove. Rather, the scope of the present invention is defined by the claims.

## Claims

1. A computer implemented method for electrophysiological mapping, comprising:
receiving an electrophysiological (EP) map (202, 212, 222, 400) of at least a portion of a surface of a cardiac chamber, the EP map comprising multiple EP values (306, 406, 408) overlayed at multiple respective positions on the surface, wherein each EP value is a local activation time (LAT) value and is derived within a window of interest (WOI) (310) on an electrogram acquired at the respective position, and wherein the WOI is a portion of a cardiac cycle used for LAT estimation;
identifying a clinical input marked on the EP map by a user using an input device (26, 37), wherein the clinical input comprises an indication of one or more activation paths (204, 214) electronically drawn on the EP map by the user using the input device; and
automatically adjusting one or more of the LAT values so that the LAT values monotonically increase along the one or more activation paths, wherein adjusting the one or more LAT values comprises adjusting the WOI on the electrograms acquired at the respective positions and recalculating the LAT values based on the adjusted WOI.

2. The method according to claim 1, and comprising adjusting one or more of the positions to be consistent with the clinical input.

3. The method according to claim 1, wherein the clinical input further comprises an indication of one or more regions (224) on the EP map.

4. The method according to claim 3, wherein the one or more regions are electronically drawn on the EP map using a touchscreen (26) displaying the EP map.

5. The method according to claim 4, wherein adjusting the EP values further comprises adjusting a level-of-confidence threshold of the EP values in the one or more regions.

6. The method according to claim 1, wherein identifying the clinical input comprises applying predefined inclusion criteria to determine which of the EP values is to be considered in relation with the identified clinical input.

7. A system (21) for electrophysiological mapping, comprising:
a memory (33) configured to store an electrophysiological (EP) map (202, 212, 222, 400) of at least a portion of a surface of a cardiac chamber, the EP map comprising multiple EP values (306, 406, 408) overlayed at multiple respective positions on the surface, wherein each EP value is a local activation time (LAT) value and is derived within a window of interest (WOI) (310) on an electrogram acquired at the respective position, and wherein the WOI is a portion of a cardiac cycle used for LAT estimation; and
a processor (28), which is configured to:
receive a clinical input marked on the EP map by a user using an input device, wherein the clinical input comprises an indication of one or more activation paths (204, 214) electronically drawn on the EP map by the user using the input device; and
automatically adjust one or more of the LAT values so that the LAT values monotonically increase along the one or more activation paths, wherein the processor is configured to adjust the LAT values by adjusting the WOI on the electrograms acquired at the respective positions and recalculating the LAT values based on the adjusted WOI.

8. The system according to claim 7, wherein the processor is further configured to adjust one or more of the positions to be consistent with the clinical input.

9. The method according to claim 1 or the system according to claim 7, wherein the one or more activation paths are electronically drawn on the EP map using a touchscreen (26) displaying the EP map.

10. The system according to claim 7, wherein the clinical input further comprises an indication of one or more regions (224) on the EP map.

11. The method according to claim 3 or the system according to claim 10, wherein the one or more regions are electronically drawn on the EP map using a touchscreen (26) displaying the EP map.

12. The system according to claim 11, wherein the processor is configured to adjust the EP values by adjusting a level-of-confidence threshold of the EP values in the one or more regions.

13. The system according to claim 7, wherein the processor is configured to identify the clinical input by applying predefined inclusion criteria to determine which of the EP values is to be considered in relation with the identified clinical input.

14. The method according to claim 1 or the system according to claim 7, wherein the input device is one of a touchscreen (26), a computer mouse, and a trackball (37).

## Patentansprüche

1. Computerimplementiertes Verfahren zum elektrophysiologischen Kartieren, umfassend:
Empfangen einer elektrophysiologischen (EP) Karte (202, 212, 222, 400) von mindestens einem Abschnitt einer Oberfläche einer Herzkammer, die EP-Karte umfassend mehrere EP-Werte (306,406,408), die an mehreren jeweiligen Positionen auf der Oberfläche überlagert sind, wobei jeder EP-Wert ein Wert einer lokalen Aktivierungszeit (LAT) ist und innerhalb eines interessierenden Fensters (WOI) (310) auf einem an der jeweiligen Position erfassten Elektrogramm abgeleitet wird, und wobei das WOI ein Abschnitt eines Herzzyklus ist, der für eine LAT-Schätzung verwendet wird;
Identifizieren einer durch einen Benutzer unter Verwendung einer Eingabevorrichtung (26, 37) auf der EP-Karte markierten klinischen Eingabe, wobei die klinische Eingabe eine Angabe eines oder mehrerer Aktivierungspfade (204, 214) umfasst, die auf der EP-Karte durch den Benutzer unter Verwendung der Eingabevorrichtung elektronisch eingezeichnet sind; und
automatisches Anpassen eines oder mehrerer der LAT-Werte, sodass die LAT-Werte entlang des einen oder der mehreren Aktivierungspfade monoton ansteigen, wobei das Anpassen des einen oder der mehreren LAT-Werte das Anpassen des WOI auf den an den jeweiligen Positionen erfassten Elektrogrammen und ein Neuberechnen der LAT-Werte basierend auf dem angepassten WOI umfasst.

2. Verfahren nach Anspruch 1 und umfassend das Anpassen einer oder mehrerer der Positionen, um mit der klinischen Eingabe übereinzustimmen.

3. Verfahren nach Anspruch 1, wobei die klinische Eingabe ferner eine Angabe einer oder mehrerer Regionen (224) auf der EP-Karte umfasst.

4. Verfahren nach Anspruch 3, wobei die eine oder die mehreren Regionen auf der EP-Karte unter Verwendung eines Touchscreens (26), der die EP-Karte anzeigt, elektronisch eingezeichnet sind.

5. Verfahren nach Anspruch 4, wobei das Anpassen der EP-Werte ferner das Anpassen einer Konfidenzniveauschwelle der EP-Werte in der einen oder den mehreren Regionen umfasst.

6. Verfahren nach Anspruch 1, wobei das Identifizieren der klinischen Eingabe das Anwenden vorgegebener Einschlusskriterien umfasst, um zu bestimmen, welcher der EP-Werte in Bezug auf die identifizierte klinische Eingabe zu berücksichtigen ist.

7. System (21) zum elektrophysiologischen Kartieren, umfassend:
einen Speicher (33), der konfiguriert ist, um eine elektrophysiologische (EP) Karte (202, 212, 222, 400) von mindestens einem Abschnitt einer Oberfläche einer Herzkammer zu speichern, die EP-Karte umfassend mehrere EP-Werte (306, 406, 408), die an mehreren jeweiligen Positionen auf der Oberfläche überlagert sind, wobei jeder EP-Wert ein Wert der lokalen Aktivierungszeit (LAT) ist und innerhalb eines interessierenden Fensters (WOI) (310) auf einem an der jeweiligen Position erfassten Elektrogramm abgeleitet wird, und wobei das WOI ein Abschnitt eines Herzzyklus ist, der für die LAT-Schätzung verwendet wird; und
einen Prozessor (28), der konfiguriert ist zum:
Empfangen einer durch einen Benutzer unter Verwendung einer Eingabevorrichtung auf der EP-Karte markierten klinischen Eingabe, wobei die klinische Eingabe eine Angabe eines oder mehrerer Aktivierungspfade (204, 214) umfasst, die auf der EP-Karte durch den Benutzer unter Verwendung der Eingabevorrichtung elektronisch eingezeichnet sind; und
automatischen Anpassen eines oder mehrerer der LAT-Werte, sodass die LAT-Werte entlang des einen oder der mehreren Aktivierungspfade monoton ansteigen, wobei der Prozessor konfiguriert ist, um die LAT-Werte durch Anpassen des WOI auf den an den jeweiligen Positionen erfassten Elektrogrammen anzupassen und die LAT-Werte basierend auf dem angepassten WOI neu zu berechnen.

8. System nach Anspruch 7, wobei der Prozessor ferner konfiguriert ist, um eine oder mehrere der Positionen anzupassen, um mit der klinischen Eingabe übereinzustimmen.

9. Verfahren nach Anspruch 1 oder System nach Anspruch 7, wobei der eine oder die mehreren Aktivierungspfade auf der EP-Karte unter Verwendung eines Touchscreens (26), der die EP-Karte anzeigt, elektronisch eingezeichnet sind.

10. System nach Anspruch 7, wobei die klinische Eingabe ferner eine Angabe einer oder mehrerer Regionen (224) auf der EP-Karte umfasst.

11. Verfahren nach Anspruch 3 oder System nach Anspruch 10, wobei die eine oder die mehreren Regionen auf der EP-Karte unter Verwendung eines Touchscreens (26), der die EP-Karte anzeigt, elektronisch eingezeichnet sind.

12. System nach Anspruch 11, wobei der Prozessor konfiguriert ist, um die EP-Werte durch Anpassen einer Konfidenzniveauschwelle der EP-Werte in der einen oder den mehreren Regionen anzupassen.

13. System nach Anspruch 7, wobei der Prozessor konfiguriert ist, um die klinische Eingabe durch Anwenden vorgegebener Einschlusskriterien zu identifizieren, um zu bestimmen, welcher der EP-Werte in Bezug auf die identifizierte klinische Eingabe zu berücksichtigen ist.

14. Verfahren nach Anspruch 1 oder System nach Anspruch 7, wobei die Eingabevorrichtung eines von einem Touchscreen (26), einer Computermaus und einem Trackball (37) ist.

## Revendications

1. Procédé mis en œuvre par ordinateur pour une cartographie électrophysiologique, comprenant :
la réception d'une carte électrophysiologique (EP) (202, 212, 222, 400) d'au moins une partie d'une surface d'une cavité cardiaque, la carte EP comprenant de multiples valeurs EP (306,406,408) superposées au niveau de multiples positions respectives sur la surface, dans lequel chaque valeur EP est une valeur de temps d'activation locale (LAT) et est dérivée dans une fenêtre d'intérêt (WOI) (310) sur un électrogramme acquis au niveau de la position respective, et dans lequel la WOI est une partie d'un cycle cardiaque utilisée pour l'estimation de LAT ;
l'identification d'une entrée clinique marquée sur la carte EP par un utilisateur à l'aide d'un dispositif d'entrée (26, 37), dans lequel l'entrée clinique comprend une indication d'une ou plusieurs voies d'activation (204, 214) tracées électroniquement sur la carte EP par l'utilisateur à l'aide du dispositif d'entrée ; et
l'ajustement automatique d'une ou plusieurs des valeurs de LAT de sorte que les valeurs de LAT augmentent de façon monotone le long de la ou des voies d'activation, dans lequel l'ajustement de la ou des valeurs de LAT comprend l'ajustement de la WOI sur les électrogrammes acquis au niveau des positions respectives et le recalcul des valeurs de LAT sur la base de la WOI ajustée.

2. Procédé selon la revendication 1, et comprenant l'ajustement d'une ou plusieurs des positions pour qu'elles soient cohérentes avec l'entrée clinique.

3. Procédé selon la revendication 1, dans lequel l'entrée clinique comprend en outre une indication d'une ou plusieurs régions (224) sur la carte EP.

4. Procédé selon la revendication 3, dans lequel la ou les régions sont tracées électroniquement sur la carte EP à l'aide d'un écran tactile (26) affichant la carte EP.

5. Procédé selon la revendication 4, dans lequel l'ajustement des valeurs EP comprend en outre l'ajustement d'un seuil de niveau de confiance des valeurs EP dans la ou les régions.

6. Procédé selon la revendication 1, dans lequel l'identification de l'entrée clinique comprend l'application de critères d'inclusion prédéfinis pour déterminer laquelle des valeurs EP doit être prise en considération par rapport à l'entrée clinique identifiée.

7. Système (21) pour la cartographie électrophysiologique, comprenant :
une mémoire (33) configurée pour stocker une carte électrophysiologique (EP) (202, 212, 222, 400) d'au moins une partie d'une surface d'une cavité cardiaque, la carte EP comprenant de multiples valeurs EP (306, 406, 408) superposées au niveau de multiples positions respectives sur la surface, dans lequel chaque valeur EP est une valeur de temps d'activation locale (LAT) et est dérivée dans une fenêtre d'intérêt (WOI) (310) sur un électrogramme acquis au niveau de la position respective, et dans lequel la WOI est une partie d'un cycle cardiaque utilisée pour l'estimation de LAT ; et
un processeur (28) qui est configuré pour :
recevoir une entrée clinique marquée sur la carte EP par un utilisateur à l'aide d'un dispositif d'entrée, dans lequel l'entrée clinique comprend une indication d'une ou plusieurs voies d'activation (204, 214) tracées électroniquement sur la carte EP par l'utilisateur à l'aide du dispositif d'entrée ; et
ajuster automatiquement une ou plusieurs des valeurs de LAT de sorte que les valeurs de LAT augmentent de façon monotone le long de la ou des voies d'activation, dans lequel le processeur est configuré pour ajuster les valeurs de LAT en ajustant la WOI sur les électrogrammes acquis au niveau des positions respectives et en recalculant les valeurs de LAT sur la base de la WOI ajustée

8. Système selon la revendication 7, dans lequel le processeur est en outre configuré pour ajuster une ou plusieurs des positions pour qu'elles soient cohérentes avec l'entrée clinique.

9. Procédé selon la revendication 1 ou système selon la revendication 7, dans lequel la ou les voies d'activation sont tracées électroniquement sur la carte EP à l'aide d'un écran tactile (26) affichant la carte EP.

10. Système selon la revendication 7, dans lequel l'entrée clinique comprend en outre une indication d'une ou plusieurs régions (224) sur la carte EP.

11. Procédé selon la revendication 3 ou système selon la revendication 10, dans lequel la ou les régions sont tracées électroniquement sur la carte EP à l'aide d'un écran tactile (26) affichant la carte EP.

12. Système selon la revendication 11, dans lequel le processeur est configuré pour ajuster les valeurs EP en ajustant un seuil de niveau de confiance des valeurs EP dans la ou les régions.

13. Système selon la revendication 7, dans lequel le processeur est configuré pour identifier l'entrée clinique en appliquant des critères d'inclusion prédéfinis pour déterminer laquelle des valeurs EP doit être prise en considération par rapport à l'entrée clinique identifiée.

14. Procédé selon la revendication 1 ou système selon la revendication 7, dans lequel le dispositif d'entrée est l'un parmi un écran tactile (26), une souris d'ordinateur et une boule de commande (37).
